# EUROPEAN PATENT APPLICATION

(11) **EP 4 644 868 A1**
(43) Date of publication of application: **05.11.2025**
(21) Application number: 23911434.1
(22) Date of filing: 16.11.2023
(51) Int. Cl.: G01N 21/33, G01N 21/61

(54) **ANALYSIS DEVICE, ANALYSIS METHOD, AND PROGRAM**

(30) Priority: 27.12.2022 JP 2022210658
(71) Applicant: HORIBA, Ltd., Minami-ku Kyoto-shi Kyoto 601-8510 (JP)
(72) Inventor: OKUDA, Tatsuya, Kyoto-shi, Kyoto 601-8510 (JP); OTSUKA, Gaku, Kyoto-shi, Kyoto 601-8510 (JP); MIZUMOTO, Kazunori, Kyoto-shi, Kyoto 601-8510 (JP); NAGASAWA, Kenya, Kyoto-shi, Kyoto 601-8510 (JP)
(74) Representative: Müller Hoffmann & Partner
(86) International application number: PCT/JP2023/041258
(87) International publication number: WO 2024/142652

(57) **Abstract**

To suppress variation in characteristics of an device for outputting ultraviolet light and to decrease replacement frequency of the device, an analysis device (100) includes a measurement cell (1), an LED light source (3), and a control unit (9). An analysis target gas is introduced into the measurement cell (1). The LED light source (3) outputs ultraviolet light (L) toward the measurement cell (1). The control unit (9) supplies a constant first current (I₁) to the LED light source (3), and when intensity of the ultraviolet light (L) output from the LED light source (3) supplied with the first current (I₁) decreases to a predetermined threshold value (Th) or lower, it supplies the LED light source (3) with a second current (I₂) larger than the first current (I₁).

## Description

### TECHNICAL FIELD

The present invention relates to an analysis device and analysis method for analyzing an analysis target gas by using ultraviolet light, and a program that causes a computer to perform this analysis method.

### BACKGROUND ART

There is known a device that emits ultraviolet light to an analysis target gas having absorption characteristic in an ultraviolet wavelength region, and analyzes the analysis target gas based on intensity of the ultraviolet light after passing through the analysis target gas. In such a device using ultraviolet light, a solid-state light emitting device (such as an LED device) may be used as a light source that outputs ultraviolet light (see, for example, Patent Literature 1).

### PRIOR ART LITERATURE

### PATENT LITERATURE

Patent Literature 1: Japanese Laid-open Patent Publication 2015-59784

### DISCLOSURE OF THE INVENTION

### PROBLEM TO BE SOLVED BY THE INVENTION

In the above Patent Citation 1, during analysis of the analysis target gas, the current supplied to the solid-state light emitting device is corrected to maintain intensity of the ultraviolet light generated from the solid-state light emitting device to be constant. The intensity of the ultraviolet light generated from the solid-state light emitting device decreases over time, and hence it is necessary to continuously increase the current supplied to the solid-state light emitting device, in order to maintain the intensity of the generated ultraviolet light to be constant.

When continuously increasing the current supplied to the solid-state light emitting device, the solid-state light emitting device generates heat, and temperature of the solid-state light emitting device may increase gradually. As a result, the life of the solid-state light emitting device is shortened, and it may be necessary to frequently replace the solid-state light emitting device. Replacement of the solid-state light emitting device requires to stop the analysis device, and hence frequent replacement of the solid-state light emitting device may be undesirable depending on operational status of the device, e.g., when the analysis target gas is continuously analyzed.

In addition, if temperature of the solid-state light emitting device increases, characteristics of the solid-state light emitting device may be changed, e.g., spectrum of the ultraviolet light output from the solid-state light emitting device may be changed. As a result, for example, the intensity of the ultraviolet light after passing through the analysis target gas having the same concentration may be changed, which may affect a result of the analysis of the analysis target gas using the ultraviolet light.

It is an object of the present invention to suppress variation in characteristics of an device for outputting ultraviolet light, and to decrease replacement frequency of the device, in a device that analyzes an analysis target gas by using ultraviolet light.

### MEANS FOR SOLVING PROBLEMS

Hereinafter, a plurality of embodiments are described as means for solving the problem. These embodiments can be arbitrarily combined as necessary.

An analysis device according to one aspect of the present invention is a device that analyzes an analysis target gas by using ultraviolet light. The analysis device includes a measurement cell, a LED light source, and a control unit. The analysis target gas is introduced into the measurement cell. The LED light source is configured to output ultraviolet light toward the measurement cell. The control unit is configured to control the LED light source. In this analysis device, the control unit supplies a constant first current to the LED light source, and when intensity of the ultraviolet light output from the LED light source supplied with the first current decreases to a predetermined threshold value or lower, the control unit supplies the LED light source with a second current larger than the first current.

In the above analysis device, unless the intensity of the ultraviolet light output from the LED light source becomes the predetermined threshold value (e.g., intensity at which the analysis target gas cannot be analyzed accurately) or lower, the control unit supplies the constant first current to the LED light source. In this way, temperature increase of the LED light source due to the supplied current can be suppressed, and a change in characteristics of the LED light source due to the temperature increase can be suppressed. As a result, the above analysis device can analyze the analysis target gas stably for a long time.

In addition, since the temperature increase of the LED light source due to the supplied current can be suppressed, the life of the LED light source can be extended. Further, the current supplied to the LED light source is increased when the intensity of the ultraviolet light output from the LED light source decreases to the predetermined threshold value or lower, and hence the intensity of the ultraviolet light can be increased again without replacing the LED light source to continue the analysis. In this way, replacement frequency of the LED light source can be reduced. As a result, analysis of the analysis target gas can be continued for a long time without stopping the analysis device.

The above analysis device may further include an input unit that is configured to receive a user's predetermined operation. In this case, when the predetermined operation is performed on the input unit, the control unit may change the current supplied to the LED light source from the first current to the second current. In this way, the current supplied to the LED light source can be increased at user's desired timing. As a result, the current supplied to the LED light source can be increased at timing that does not affect analysis of the analysis target gas.

The above analysis device may further include an alarm generation unit that is configured to generate an alarm when the ultraviolet light output from the LED light source becomes a predetermined threshold value or lower. In this way, it can be easily recognized that the ultraviolet light from the LED light source has become the predetermined threshold value or lower.

The above analysis device may further include an introducing unit that is configured to alternately introduce the analysis target gas and a reference gas to the measurement cell at a predetermined period. In this way, detection of the ultraviolet light after passing through the analysis target gas and detection of the ultraviolet light after passing through the reference gas can be performed by a single detection unit. In addition, an influence to analysis of the analysis target gas caused by an interfering component contained in the gas introduced into the measurement cell can be reduced.

An analysis method according to another aspect of the present invention is a method of analyzing an analysis target gas by using ultraviolet light. This analysis method includes:
introducing the analysis target gas into a measurement cell;
supplying a constant first current to an LED light source configured to output the ultraviolet light to output the ultraviolet light from the LED light source toward the measurement cell; and
supplying the LED light source with a second current larger than the first current, when intensity of the ultraviolet light output from the LED light source supplied with the first current decreases to a predetermined threshold value or lower.

In the above analysis method, unless the intensity of the ultraviolet light output from the LED light source becomes the predetermined threshold value or lower, the constant first current is supplied to the LED light source. In this way, temperature increase of the LED light source due to the supplied current can be suppressed, and a change in characteristics of the LED light source due to the temperature increase can be suppressed. As a result, the analysis target gas can be analyzed stably for a long time.

In addition, since the temperature increase of the LED light source due to the supplied current can be suppressed, the life of the LED light source can be extended. Further, the current supplied to the LED light source is increased when the intensity of the ultraviolet light output from the LED light source decreases to the predetermined threshold value or lower, and hence the intensity of the ultraviolet light can be increased again without replacing the LED light source to continue the analysis. In this way, replacement frequency of the LED light source can be reduced. As a result, analysis of the analysis target gas can be continued for a long time without stopping the analysis device.

A program according to still another aspect of the present invention is a program that causes a computer to perform the above analysis method.

### EFFECTS OF INVENTION

It is possible to suppress variation in characteristics of the LED light source for outputting ultraviolet light, and to reduce replacement frequency of the device.

### BRIEF DESCRIPTION OF DRAWINGS

[Fig. 1] A diagram illustrating a structure of an analysis device.
[Fig. 2] A diagram illustrating an example of a light amount history display screen.
[Fig. 3] A flowchart illustrating an analysis operation of an analysis target gas.
[Fig. 4] A diagram illustrating an example of temporal changes of a current value supplied to an LED light source and intensity of the ultraviolet light, in the analysis operation.
[Fig. 5] A diagram illustrating an example of a present light amount display screen.
[Fig. 6] A diagram illustrating an example of a current value setting screen.

### DESCRIPTION OF EMBODIMENTS

### 1. First Embodiment

### (1) Outline

Hereinafter, an analysis device 100 according to a first embodiment is described. The analysis device 100 is a device that analyzes a specific gas (referred to as an analysis target gas), which is contained in an atmosphere to be measured such as air and absorbs light having a wavelength in the ultraviolet region. Specifically, the analysis device 100 passes ultraviolet light L through a gas (referred to as a sample gas Gs) containing the analysis target gas, and analyzes the analysis target gas based on intensity of the ultraviolet light after passing through the sample gas Gs. For instance, information about concentration of the analysis target gas contained in the sample gas is calculated as a result of the analysis. The gas to be analyzed by the analysis device 100 is ozone (O₃), for example. Note that, without limiting to ozone, the analysis target gas may be a gas such as sulfur dioxide (SO₂) that absorbs light having a wavelength in the ultraviolet region, toluene (C₇H₈), or moisture (H₂O). In addition, it is also possible to analyze a gas other than the analysis target gas, as an interference affecting gas.

### (2) Structure of Analysis Device

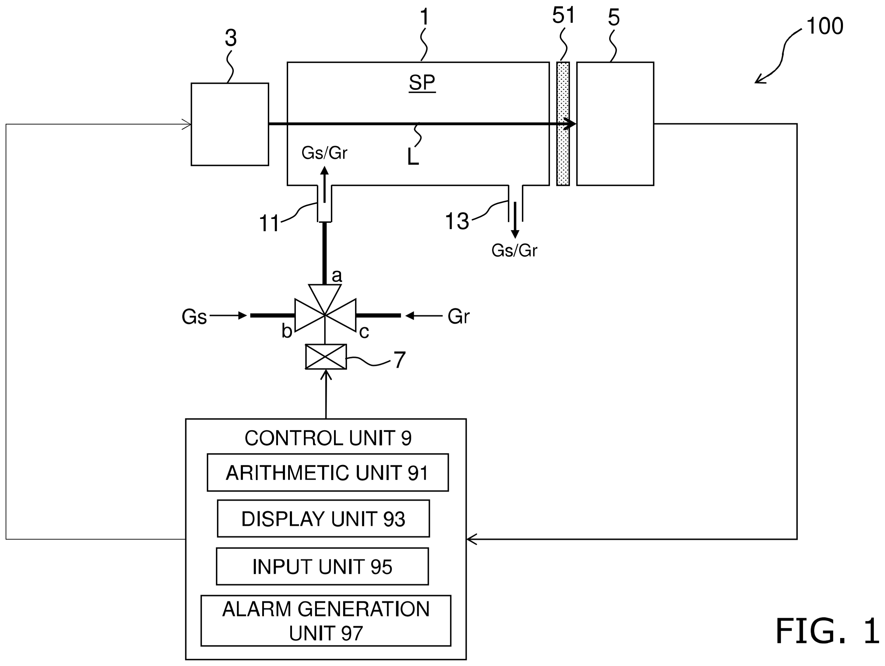

With reference to Fig. 1, a structure of the analysis device 100 is described. Fig. 1 is a diagram illustrating a structure of the analysis device. The analysis device 100 includes a measurement cell 1, an LED light source 3, a detection unit 5, an introducing unit 7, and a control unit 9. The measurement cell 1 is a member having an internal space SP. One end of the measurement cell 1 is provided with an inlet 11 connected to the internal space SP, and the other end of the same is provided with an outlet 13 connected to the internal space SP. The sample gas Gs or a reference gas Gr is introduced by the introducing unit 7 into the internal space SP of the measurement cell 1 through the inlet 11. The sample gas Gs or the reference gas Gr introduced into the internal space SP is discharged through the outlet 13.

If the analysis target gas is ozone, the measurement cell 1 is made of a material such as glass that is not react with ozone. If the measurement cell 1 is made of a transparent material such as glass, a metal thin film (such as a sputtering film of chromium (Cr)) is formed on the outer surface of the measurement cell 1. In this way, the ultraviolet light L output from the LED light source 3 is reflected by the surface of the measurement cell 1, and is suppressed from leaking from the measurement cell 1.

The LED light source 3 is a light emitting diode (LED) device that is disposed on one end side of the measurement cell 1 to output the ultraviolet light L toward the measurement cell 1. The ultraviolet light L contains at least a light component having a wavelength that is absorbed by the analysis target gas. If the analysis target gas is ozone, the ultraviolet light L contains at least a light component having a wavelength near 254 nm, for example.

The detection unit 5 is disposed on the side of the measurement cell 1 opposite to the side on which the LED light source 3 is disposed, so as to detect the ultraviolet light L that is output from the LED light source 3 and passes through the internal space SP of the measurement cell 1. The detection unit 5 is, for example, an device such as a silicon photodiode that can detect the ultraviolet light L.

As illustrated in Fig. 1, an optical filter 51 may be disposed between the measurement cell 1 and the detection unit 5. The optical filter 51 transmits only the light component of the ultraviolet light L, which has a wavelength that the analysis target gas absorbs. In this way, only the light component having a wavelength that the analysis target gas absorbs can be detected by the detection unit 5, and hence it is possible to calculate a result of analysis that is not affected by other light components of the ultraviolet light L.

The introducing unit 7 is, for example, a three-way electromagnetic valve having three gas ports a, b, and c. The gas port a is connected to the inlet 11 of the measurement cell 1. The gas port b is connected to a gas line into which the sample gas Gs is introduced. Specifically, the gas port b is connected to a device for collecting the sample gas Gs (e.g., a sampling probe). A collecting flow rate of the sample gas Gs can be 0.6 L/min, for example.

The gas port c is connected to a gas line into which the reference gas Gr is introduced. The introducing unit 7 switches between a state where the gas can flow between the gas port a and the gas port b, and a state where the gas can flow between the gas port a and the gas port c, alternately at a predetermined period in accordance with a signal from the control unit 9.

In the state where the gas can flow between the gas port a and the gas port b, the sample gas Gs is introduced into the internal space SP of the measurement cell 1. In contrast, in the state where the gas can flow between the gas port a and the gas port c, the reference gas Gr is introduced into the internal space SP of the measurement cell 1. As a result, by the above switching, the introducing unit 7 can alternately introduce the sample gas Gs and the reference gas Gr into the internal space SP of the measurement cell 1 at a predetermined period.

In this way, since the sample gas Gs and the reference gas Gr can be introduced alternately at a predetermined period into the internal space SP of the measurement cell 1, it is not necessary to separately dispose the detection unit for measuring intensity of the ultraviolet light L after passing through the sample gas Gs, and the detection unit for measuring intensity of the ultraviolet light L after passing through the reference gas Gr. In other words, detection of the ultraviolet light L after passing through the sample gas Gs and detection of the ultraviolet light L after passing through the reference gas Gr can both be performed with the single detection unit 5.

The reference gas Gr is a gas after removing the analysis target gas from the sample gas Gs. If the analysis target gas is ozone, the reference gas Gr can be produced by passing the sample gas Gs through an ozone decomposition device having a catalyst or the like that removes ozone, for example.

In the analysis device 100, the above gas line is constituted using a piping member (e.g., a coupling) made of a fluorocarbon resin (such as PTFE).

The control unit 9 is a computer system including a CPU, a storage device (such as a RAM, a ROM, an HDD, or an SSD), various interfaces, and a display. The control unit 9 includes an arithmetic unit 91, a display unit 93, an input unit 95, and an alarm generation unit 97.

The arithmetic unit 91 is constituted of the CPU, the storage device, and the various interfaces that are included in the control unit 9, and perform processing related to control of the analysis device 100. Specifically, the arithmetic unit 91 controls the introducing unit 7 to switch between the state where the gas can flow between the gas port a and the gas port b (the state where the sample gas Gs is introduced into the internal space SP of the measurement cell 1), and the state where the gas can flow between the gas port a and the gas port c (the state where the reference gas Gr is introduced into the internal space SP), at a predetermined period.

The arithmetic unit 91 controls the current supplied to the LED light source 3. The arithmetic unit 91 analyzes the analysis target gas based on a signal output from the detection unit 5. For instance, the arithmetic unit 91 calculates concentration of the analysis target gas contained in the sample gas Gs, based on the signal output from the detection unit 5.

The arithmetic unit 91 may realize a part or a whole of the above control by executing a program stored in the storage device. In addition, the arithmetic unit 91 may realize a part or a whole of the above control by hardware.

The display unit 93 is a display of the control unit 9, and displays a display related to the control of the analysis device 100, a display related to analysis of the analysis target gas, a result of the analysis of the analysis target gas, and the like. For instance, the display unit 93 can display a result (measured value) of the analysis of the analysis target gas, various alarms, an alarm generation history, a calibration history of the analysis device 100, present time, and the like. The display unit 93 is, for example, a display such as a liquid crystal display, or an organic EL display.

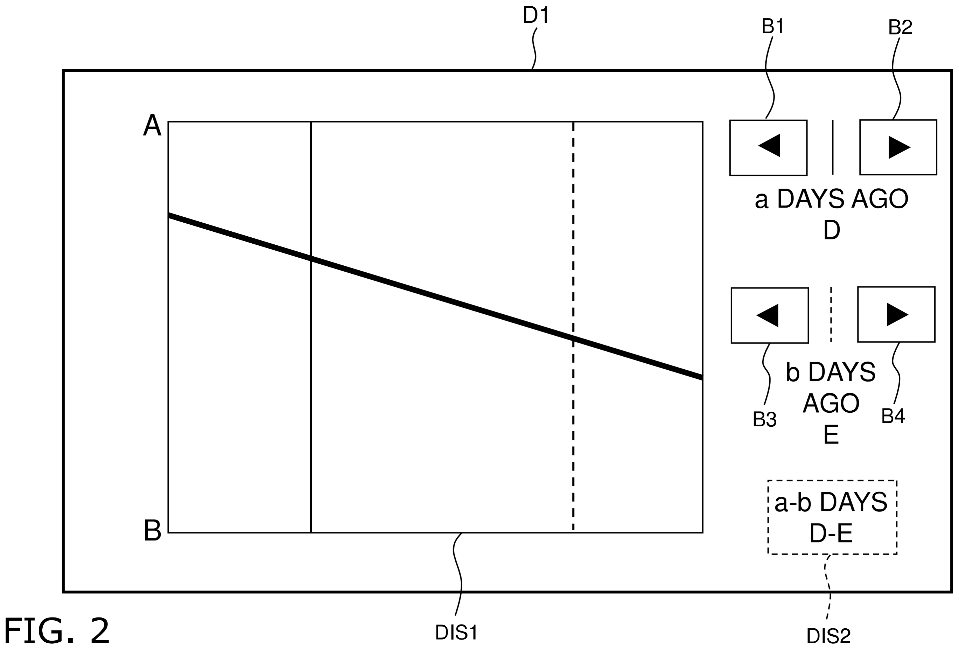

The display unit 93 can display a light amount history display screen D1 as illustrated in Fig. 2. Fig. 2 is a diagram illustrating an example of the light amount history display screen. The light amount history display screen D1 includes a graph display section DIS1 that displays a graph of a temporal change of light amount (intensity) of the ultraviolet light L output from the LED light source 3. In the graph display section DIS1, the horizontal axis represents time, and the vertical axis represents the light amount. The minimum value of the vertical axis is set to a predetermined threshold value Th that will be described later. In the example illustrated in Fig. 2, the predetermined threshold value Th is set to "B".

In the light amount history display screen D1, a change of the light amount between two reference time points (referred to as a first reference time point and a second reference time point) can be displayed. Note that, in the graph display section DIS1 illustrated in Fig. 2, the first reference time point is shown by a solid vertical line, and the second reference time point is shown by a broken vertical line.

The light amount history display screen D1 has a first button B1 and a second button B2 for setting the first reference time point, and a third button B3 and a fourth button B4 for setting the second reference time point, and a change amount display section DIS2 that displays the change amount of the light amount. Below the first button B1 and the second button B2, a set value of the first reference time point (set as "a days ago" in Fig. 2) and the light amount at the first reference time point ("D" in Fig. 2) are displayed. In addition, below the third button B3 and the fourth button B4, a set value of the second reference time point (set as "b days ago" in Fig. 2) and the light amount at the first reference time point ("E" in Fig. 2) are displayed.

The change amount display section DIS2 displays a time period as a difference between the first reference time point and the second reference time point ("a-b days" in Fig. 2), and the change of the light amount from the first reference time point to the second reference day ("D-E" in Fig. 2).

Since the display unit 93 displays the light amount history display screen D1 as illustrated in Fig. 2, it is possible to visually check a light amount variation state (e.g., a rate of decrease in the light amount) of the ultraviolet light L output from the LED light source 3.

The input unit 95 is, for example, an input device such as a touch panel, a keyboard, or a mouse, provided to the display of the control unit 9. The input unit 95 accepts a user's predetermined operation (e.g., a touch of the touch panel, an input by the keyboard, or a click of a mouse button). The input unit 95 is not limited to the above input device such as a touch panel, a keyboard, or a mouse. For instance, the input unit 95 may be another computer connected to the control unit 9.

In this embodiment, for example, the intensity of the ultraviolet light L output from the LED light source 3 becomes the predetermined threshold value Th or less, and when user recognizes this fact and performs the predetermined operation on the input unit 95, the arithmetic unit 91 increases the current value to be supplied to the LED light source 3. In this way, the analysis device 100 can adjust the current value supplied to the LED light source 3 at user's desired timing.

The control unit 9 can set a measurement range of the analysis target gas in accordance with a user's operation using the input unit 95. The control unit 9 can set the measurement range to 0 to 0.1 ppm, 0 to 0.2 ppm, 0 to 0.5 ppm, 0 to 1.0 ppm, 0 to 2.0 ppm, 0 to 5.0 ppm, or 0 to 10.0 ppm, for example.

When the ultraviolet light L output from the LED light source 3 has become the predetermined threshold value Th or less, the alarm generation unit 97 generates an alarm notifying that the ultraviolet light L output from the LED light source 3 has become the predetermined threshold value Th or less. The alarm generation unit 97 can generate an alarm displaying that the ultraviolet light L output from the LED light source 3 has become the predetermined threshold value Th or less, for example. In this case, the alarm generation unit 97 is the display of the control unit 9.

In addition, when the ultraviolet light L output from the LED light source 3 has become the predetermined threshold value Th or less, the alarm generation unit 97 may generate the alarm by generating a predetermined sound. In this case, the alarm generation unit 97 is a speaker that generates a sound, for example. Further, the alarm generation unit 97 may be an alarm lamp, and it may be possible to generate the alarm by turning on the alarm lamp, when the ultraviolet light L output from the LED light source 3 has become the predetermined threshold value Th or less. Note that the plurality of forms of the alarm generation unit 97 described above can be arbitrarily combined.

Since the control unit 9 has the above alarm generation unit 97, it is possible to notify the user that the ultraviolet light L output from the LED light source 3 has become the predetermined threshold value Th or less, in a visual and/or auditory manner. As a result, the user can easily recognize that the ultraviolet light L has become the predetermined threshold value Th or less.

The analysis device 100 can notify alarms other than the alarm indicating that the ultraviolet light L has become the predetermined threshold value Th or less. The alarms that the analysis device 100 can notify include, for example, a zero calibration abnormality alarm indicating that zero calibration is failed, a span calibration abnormality alarm indicating that span calibration is failed, a communication abnormality alarm indicating that communication between the control unit 9 and outside is abnormal, a telemeter failure alarm related to an abnormality of a telemeter, a device inside temperature abnormality alarm indicating that temperature inside the analysis device 100 is abnormal, a cell temperature abnormality alarm indicating that temperature of the measurement cell 1 is abnormal, an ozone decomposition device temperature abnormality alarm indicating that temperature of the ozone decomposition device is abnormal, an atmospheric pressure abnormality alarm indicating that a measured value of an atmospheric pressure sensor is abnormal, a sample pressure abnormality alarm indicating that an absorption pressure for absorbing the sample gas Gs is abnormal, a sample flow rate abnormality alarm indicating that a flow rate of the sample gas Gs is abnormal, a power supply abnormality alarm indicating that a power supply is abnormal, and the like.

Other than that, the analysis device 100 can notify "caution" indicating that the analysis device 100 is in a state prior to alarm, though it is not so serious as alarm. For instance, it may be possible to set a threshold value other than the above predetermined threshold value Th for the intensity of the ultraviolet light L, and when the intensity of the ultraviolet light L becomes this threshold value or lower, it may be possible to notify caution indicating that the intensity of the ultraviolet light L is approaching the minimum value (the predetermined threshold value Th).

In addition, the cautions that the analysis device 100 can notify include, for example, a zero calibration caution, a span calibration caution, a battery voltage abnormality caution related to low battery for clock, a device inside temperature caution, and the like.

Further, the analysis device 100 can also notify information indicating a state of the analysis device 100. The information that the analysis device 100 can notify include, for example, AIC information indicating that an automatic sequence is being executed, line information indicating that a measurement line is set to other than measurement, calibration execution information indicating that calibration has been executed, power supply ON information indicating that the power supply is turned on, under maintenance information indicating that the analysis device 100 is under maintenance, and the like.

The control unit 9 has various interfaces, which are a network interface, a serial interface (such as RS-232C or a USB interface), an analog input/output interface, a contact input/output interface, and the like.

The analysis device 100 having the above structure can analyze the analysis target gas in a wide concentration range described above, with a minimum detection sensitivity (2σ) of 0.5 ppb, at high speed (e.g., in 120 sec or less). The minimum detection sensitivity can be defined as a lowest concentration of the analysis target gas that can be detected by the analysis device 100.

### (3) Analysis Operation of Analysis Target Gas by Analysis Device

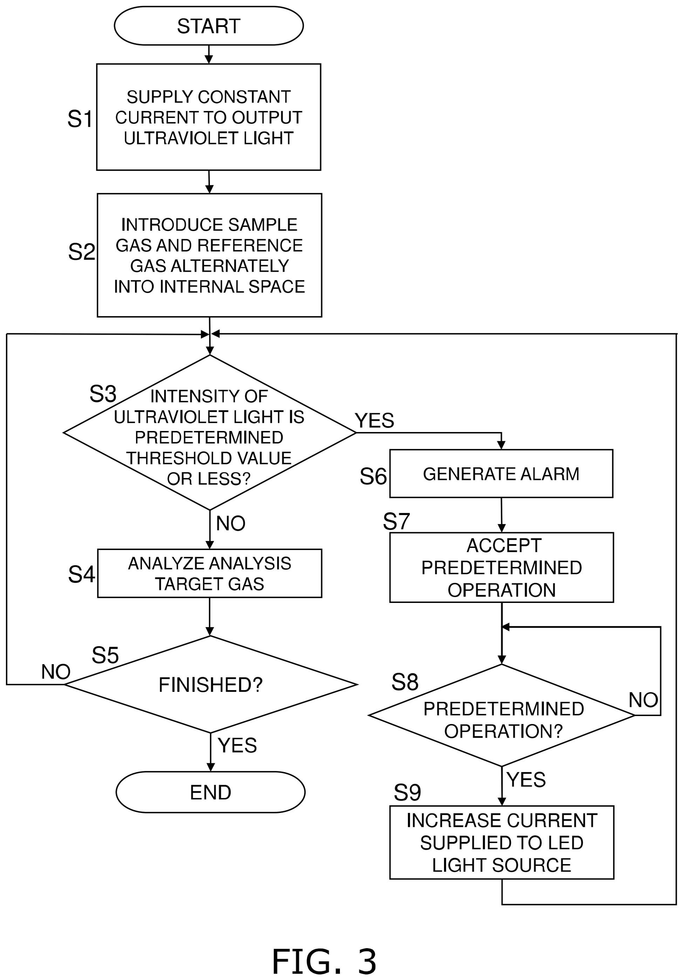

Hereinafter, an analysis operation of the analysis target gas performed by the analysis device 100 is described. When starting the analysis operation, warming up of the analysis device 100 is performed for a predetermined time period (e.g., a few hours). Then, the analysis operation is performed in accordance with a flow illustrated in Fig. 3. Fig. 3 is a flowchart illustrating the analysis operation of the analysis target gas. First, the arithmetic unit 91 of the control unit 9 supplies a constant first current I₁ to the LED light source 3 in Step S1. In this way, the LED light source 3 outputs the ultraviolet light L toward the internal space SP of the measurement cell 1.

Next, in Step S2, the arithmetic unit 91 operates the introducing unit 7 to switch between the state where the gas can flow between the gas port a and the gas port b, and the state where the gas can flow between the gas port a and the gas port c, at the predetermined period. In this way, the sample gas Gs and the reference gas Gr are alternately introduced into the internal space SP of the measurement cell 1 at the predetermined period.

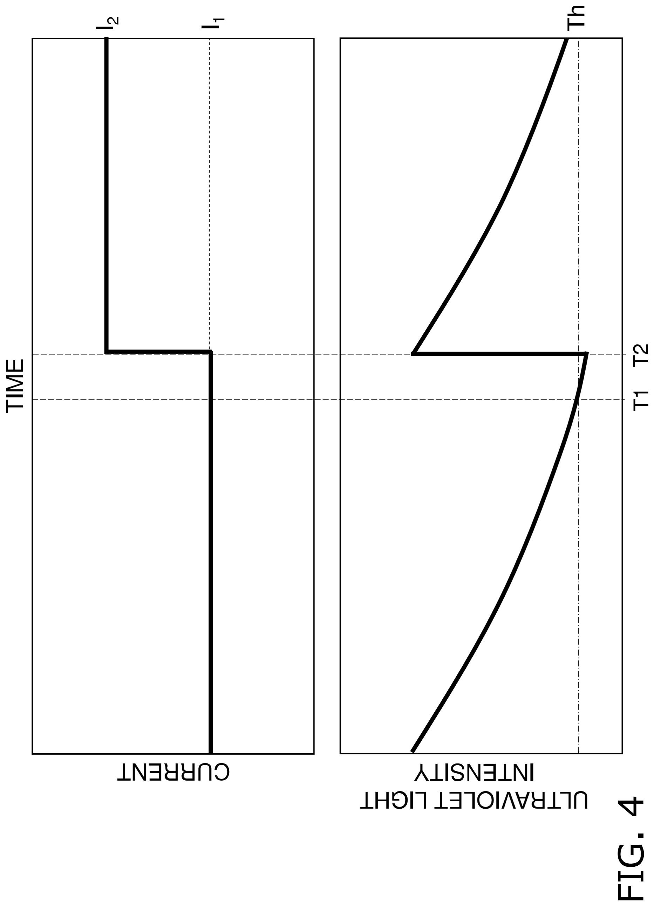

When the constant current is continuously supplied to the LED light source 3 as described above, the intensity of the ultraviolet light L output from the LED light source 3 decreases over time as illustrated in Fig. 4. If the intensity of the ultraviolet light L decreases excessively, the detection unit 5 may become unable to detect sufficient intensity of the ultraviolet light L. As a result, it may be unable to correctly analyze the analysis target gas. Fig. 4 is a diagram illustrating an example of temporal changes of the current value supplied to the LED light source and the intensity of the ultraviolet light, in the analysis operation.

Therefore, during the analysis operation of the analysis target gas, the arithmetic unit 91 determines in Step S3 whether or not the intensity of the ultraviolet light L output from the LED light source 3 has decreased to the predetermined threshold value Th or less. Here, for example, the predetermined threshold value Th can be set as the intensity of the ultraviolet light L before passing through the measurement cell 1, at which the analysis target gas contained in the sample gas Gs cannot be correctly analyzed on the basis of the intensity of the ultraviolet light L detected by the detection unit 5.

The above language "the analysis target gas cannot be correctly analyzed" can be defined, for example, as a case where the minimum detection sensitivity of the analysis device 100 for the analysis target gas becomes a predetermined value (e.g., 3 ppb) or more. In this case, the intensity of the ultraviolet light L at which the analysis target gas cannot correctly analyzed (i.e., the predetermined threshold value Th) can be set, for example, to 20% to 50% of the intensity at which the analysis target gas can be correctly analyzed (e.g., in the state where the minimum detection sensitivity is 0.5 ppb).

In addition, the intensity of the ultraviolet light L output from the LED light source 3 can be known by measuring the intensity of the ultraviolet light L before passing through the measurement cell 1, for example. Other than that, it may be also possible to theoretically calculate the intensity of the ultraviolet light L output from the LED light source 3 from the elapsed time after starting supply of the first current I₁.

When it is determined that the intensity of the ultraviolet light L output from the LED light source 3 has not decreased to the predetermined threshold value Th or less ("No" in Step S3), the arithmetic unit 91 analyzes the analysis target gas contained in the sample gas Gs in Step S4, based on the intensity of the ultraviolet light L detected by the detection unit 5.

In general, the sample gas Gs may contain a gas other than the analysis target gas, which absorbs light in the ultraviolet region (referred to as an interfering component). In this case, for example, if an absorption spectrum of the analysis target gas and an absorption spectrum of the interfering gas are partially overlapped, the intensity of the ultraviolet light L detected by the detection unit 5 is decreased by the sum of the absorption of the ultraviolet light L by the analysis target gas and the absorption of the ultraviolet light L by the interfering gas. In other words, the intensity of the ultraviolet light L detected by the detection unit 5 is affected by the interfering gas.

As described above, the sample gas Gs that contains the analysis target gas and the reference gas Gr that does not contain the same are introduced alternately at a predetermined period, into the internal space SP of the measurement cell 1 by the introducing unit 7. Therefore, in the predetermined period described above, the arithmetic unit 91 acquires the intensity of the ultraviolet light L that is partially absorbed by the sample gas Gs when the sample gas Gs is introduced into the internal space SP, and it acquires the intensity of the ultraviolet light L that is partially absorbed by the reference gas Gr when the reference gas Gr is introduced into the internal space SP. After that, the arithmetic unit 91 performs the analysis of the analysis target gas contained in the sample gas Gs, based on a difference between the intensity of the ultraviolet light L absorbed by the sample gas Gs and the intensity of the ultraviolet light L absorbed by the reference gas Gr. In this way, it is possible to reduce the influence of the interfering component contained in the sample gas Gs to the analysis of the analysis target gas.

In addition, since the intensity of the ultraviolet light L output from the LED light source decreases over time, the arithmetic unit 91 performs the analysis of the analysis target gas in consideration of the decrease of the ultraviolet light L. Specifically, for example, the arithmetic unit 91 can perform the analysis of the analysis target gas, based on a ratio between the intensity of the ultraviolet light L detected by the detection unit 5 and the intensity of the ultraviolet light L output from the LED light source.

After finishing the analysis of the analysis target gas, the arithmetic unit 91 causes the display unit 93 to display a result of the analysis of the analysis target gas. After that, unless an instruction to finish the analysis is issued ("No" in Step S5), and as long as the intensity of the ultraviolet light L output from the LED light source is more than the predetermined threshold value Th ("No" in Step S3), the analysis of the analysis target gas is continued.

On the other hand, as a result of continuing the analysis by supplying the constant first current I₁ to the LED light source 3, if the intensity of the ultraviolet light L output from the LED light source 3 has become the predetermined threshold value Th or less ("Yes" in Step S3), the arithmetic unit 91 causes the alarm generation unit 97 to generate the alarm notifying that the intensity of the ultraviolet light L has become the predetermined threshold value Th or less (Step S6).

After the alarm is generated, the arithmetic unit 91 accepts the predetermined operation using the input unit 95 in Step S7, in order to increase the current supplied to the LED light source 3. Specifically, the following operation is performed.

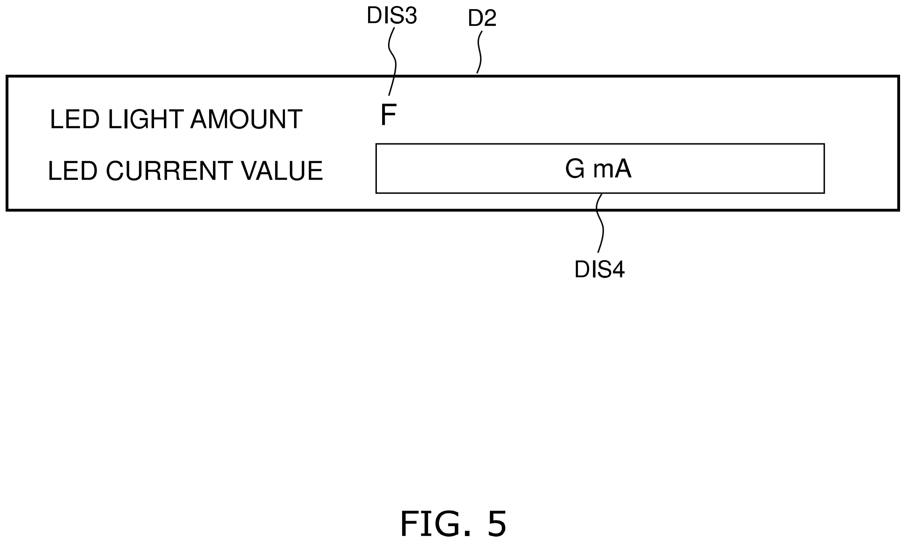

First, the arithmetic unit 91 causes the display unit 93 to display a present light amount display screen D2 as illustrated in Fig. 5. Fig. 5 is a diagram illustrating an example of the present light amount display screen. The present light amount display screen D2 has a light amount display section DIS3 that displays a present light amount of the ultraviolet light L output from the LED light source 3, and a current set value display section DIS4 that displays a present set value of the current value supplied to the LED light source 3. Note that the current set value display section DIS4 is also a button.

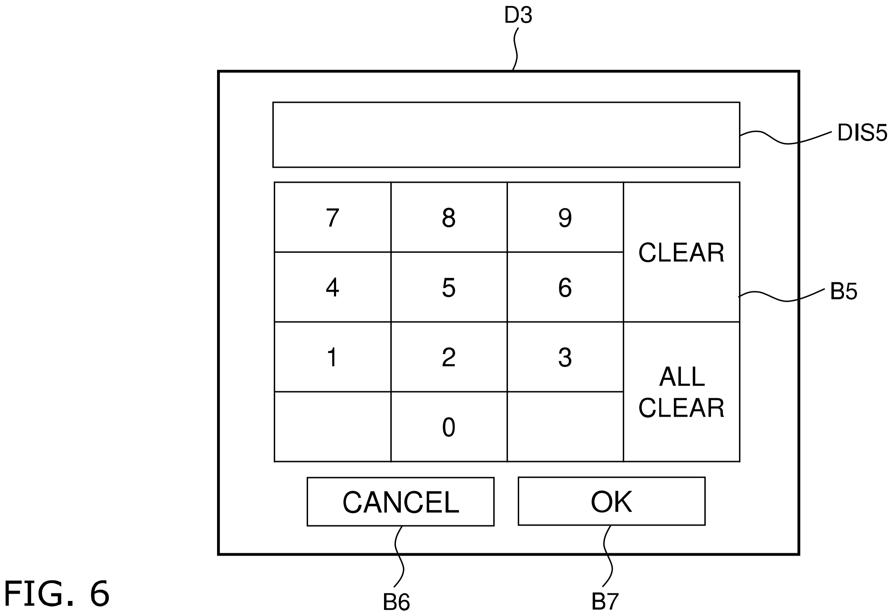

In order to set the current value to be increased, an operation of pressing the current set value display section DIS4 on the present light amount display screen D2 is performed using the input unit 95, and then the arithmetic unit 91 causes the display unit 93 to display a current value setting screen D3 as illustrated in Fig. 6. Fig. 6 is a diagram illustrating an example of the current value setting screen. The current value setting screen D3 has a numeric key section B5 for setting the current value, a set value display section DIS5 for displaying the set value that is set using the numeric key section B5, a cancel button B6 for canceling the set current value, and a setting button B7 for setting the current value to the value displayed in the set value display section DIS5.

An operation of pressing any numeric button in the numeric key section B5 is performed using the input unit 95 to set a target current value to be increased, and after that an operation of pressing the setting button B7 is performed using the input unit 95, and thus the predetermined operation for increasing the current to be supplied to the LED light source 3 is completed.

When the above predetermined operation is performed using the input unit 95 ("Yes" in Step S8), the arithmetic unit 91 increases the current to be supplied to the LED light source 3, in Step S9, from the first current I₁ to a second current I₂ having a larger current value than the first current I₁.

For instance, as illustrated in Fig. 4, if the intensity of the ultraviolet light L becomes the predetermined threshold value Th at time point T1 when the constant first current I₁ is supplied to the LED light source 3, the above alarm is generated, and then if the user notices the alarm and performs the above predetermined operation using the input unit 95 at time point T2 when the analysis device 100 is not performing the analysis, for example, the current supplied to the LED light source 3 is increased from the first current I₁ to the second current I₂. After that, the constant second current I₂ is supplied to the LED light source 3.

After the current supplied to the LED light source 3 is increased, Steps S3 to S5 described above are performed. In other words, as a result of increasing the current, if the intensity of the ultraviolet light L has become more than the predetermined threshold value Th ("Yes" in Step S3), the analysis of the analysis target gas is performed (Steps S4 to S5). On the other hand, when the current is increased, if the intensity of the ultraviolet light L has not become more than the predetermined threshold value Th ("No" in Step S3), the above Steps S6 to S9 are performed repeatedly, or maintenance such as replacing the LED light source 3 is performed.

In this way, unless the intensity of the ultraviolet light L output from the LED light source 3 becomes the predetermined threshold value Th or less, the analysis device 100 supplies the constant first current I₁ to the LED light source 3. In this way, temperature increase of the LED light source 3 due to the supplied current can be suppressed, and hence a change in characteristics of the LED light source 3 due to the temperature increase can be suppressed. As a result, the analysis target gas can be analyzed stably for a long time.

In addition, as temperature increase of the LED light source 3 due to the supplied current can be suppressed, the life of the LED light source 3 can be extended. Further, when the intensity of the ultraviolet light L output from the LED light source 3 decreases to the predetermined threshold value Th or less, the current supplied to the LED light source is increased, and hence the analysis can be continued by increasing the intensity of the ultraviolet light L again, without replacing the LED light source 3. In this way, replacement frequency of the LED light source 3 can be reduced. As a result, the analysis of the analysis target gas can be continued for a long time without stopping the analysis device.

Note that for example, if sufficient intensity of the ultraviolet light L cannot be output even if the current is increased to or close to the upper limit value of the current that can be supplied to the LED light source 3, and/or if frequent increase of the current is required as a result that the intensity of the ultraviolet light L becomes the predetermined threshold value Th or less in a short time, replacement of the LED light source 3 is performed. In general, the LED light source 3 as an LED device has a wavelength characteristic (spectrum) of the output light that is different for each device. Therefore, when the LED light source 3 is replaced, calibration (such as zero point calibration or span calibration) of the analysis device 100 is performed.

### 2. Features of Embodiment

The above embodiment is also described as follows.
(1) An analysis device (e.g., the analysis device 100) is a device for analyzing an analysis target gas by using ultraviolet light (e.g., the ultraviolet light L). The analysis device includes a measurement cell (e.g., the measurement cell 1), an LED light source (e.g., the LED light source 3), and a control unit (e.g., the control unit 9). The analysis target gas is introduced into the measurement cell. The LED light source is configured to output the ultraviolet light toward the measurement cell. The control unit is configured to control the LED light source. In this analysis device, the control unit supplies a constant first current (e.g., the first current I₁) to the LED light source, and when intensity of the ultraviolet light output from the LED light source supplied with the first current decreases to a predetermined threshold value (e.g., the predetermined threshold value Th) or less, the control unit supplies the LED light source with a second current (e.g., the second current I₂) larger than the first current.

In the above analysis device, unless the intensity of the ultraviolet light output from the LED light source becomes the predetermined threshold value or lower, the constant first current is supplied to the LED light source. In this way, temperature increase of the LED light source due to the supplied current can be suppressed, and a change in characteristics of the LED light source due to the temperature increase can be suppressed. As a result, the above analysis device can analyze the analysis target gas stably for a long time.

In addition, since the temperature increase of the LED light source due to the supplied current can be suppressed, the life of the LED light source can be extended. Further, the current supplied to the LED light source is increased when the intensity of the ultraviolet light output from the LED light source decreases to the predetermined threshold value or lower, and hence the intensity of the ultraviolet light can be increased again without replacing the LED light source to continue the analysis. In this way, replacement frequency of the LED light source can be reduced. As a result, analysis of the analysis target gas can be continued for a long time without stopping the analysis device.

(2) The analysis device of the above (1) may further include an input unit (e.g., the input unit 95) that is configured to receive a user's predetermined operation. In this case, when the predetermined operation is performed on the input unit, the control unit may change the current supplied to the LED light source from the first current to the second current. In this way, the current supplied to the LED light source can be increased at user's desired timing. As a result, the current supplied to the LED light source can be increased at timing that does not affect analysis of the analysis target gas.

(3) The analysis device of the above (1) or (2) may further include an alarm generation unit that is configured to generate an alarm when the ultraviolet light output from the LED light source becomes a predetermined threshold value or less. In this way, it can be easily recognized that the ultraviolet light from the LED light source has become the predetermined threshold value or lower.

(4) The analysis device of the above (1) to (3) may further include an introducing unit that is configured to alternately introduce the analysis target gas and a reference gas into the measurement cell at a predetermined period. In this way, detection of the ultraviolet light after passing through the analysis target gas and detection of the ultraviolet light after passing through the reference gas can be performed by a single detection unit. In addition, an influence to analysis of the analysis target gas caused by an interfering component contained in the gas introduced into the measurement cell can be reduced.

(5) An analysis method according to another aspect of the present invention is a method for analyzing an analysis target gas by using ultraviolet light. This analysis method includes:
introducing the analysis target gas into a measurement cell (e.g., Step S2);
supplying a constant first current to an LED light source configured to output the ultraviolet light, to output the ultraviolet light from the LED light source toward the measurement cell (e.g., Step S1); and
supplying the LED light source with a second current larger than the first current, when intensity of the ultraviolet light output from the LED light source supplied with the first current decreases to a predetermined threshold value or less (e.g., Step S8).

In the above analysis method, unless the intensity of the ultraviolet light output from the LED light source becomes the predetermined threshold value or lower, the constant first current is supplied to the LED light source. In this way, temperature increase of the LED light source due to the supplied current can be suppressed, and a change in characteristics of the LED light source due to the temperature increase can be suppressed. As a result, the analysis target gas can be analyzed stably for a long time.

In addition, since the temperature increase of the LED light source due to the supplied current can be suppressed, the life of the LED light source can be extended. Further, the current supplied to the LED light source is increased when the intensity of the ultraviolet light output from the LED light source decreases to the predetermined threshold value or lower, and hence the intensity of the ultraviolet light can be increased again without replacing the LED light source to continue the analysis. In this way, replacement frequency of the LED light source can be reduced.

(6) A program according to still another aspect of the present invention is a program that causes a computer to perform the analysis method of the above (5).

### 3. Other Embodiments

Although an embodiment of the present invention is described above, the present invention is not limited to the above embodiment, but can be variously modified within the scope of the invention without deviating from the spirit thereof. In particular, the plurality of embodiments and variations described in this specification can be arbitrarily combined as necessary.
(A) In the analysis operation described above using the flowchart of Fig. 3, the order of the steps in the flowchart of Fig. 3 and process details of the steps can be appropriately modified within the scope of the invention without deviating from the spirit thereof. For instance, Step S2 and Step S1 may be exchanged, so that supply of the constant first current I₁ to the LED light source 3 (i.e., output of the ultraviolet light L) may be started after introduction of the sample gas Gs/the reference gas Gr into the internal space SP of the measurement cell 1 is started.
(B) In the above first embodiment, when the predetermined operation is performed on the input unit 95, the current supplied to the LED light source 3 is increased, but this is not a limitation. For instance, when the intensity of the ultraviolet light L output from the LED light source 3 becomes the predetermined threshold value Th or less, the arithmetic unit 91 may automatically increase the current supplied to the LED light source 3.
(C) The above technique can also be applied to another analysis device, in which the sample gas Gs is introduced into the measurement cell 1, while another cell is provided to introduce or fill the reference gas Gr, and the ultraviolet light L after passing through the individual cells are detected by separate detection units.
(D) The analysis device 100 may include, in addition to the members described above, a filter for removing dust or the like from the sample gas, or a member (such as a mist trap) for removing moisture from the gas introduced into the internal space SP of the measurement cell 1, for example.

### INDUSTRIAL APPLICABILITY

The present invention can be widely applied to devices for analyzing an analysis target gas by using ultraviolet light.

### REFERENCE SIGNS LIST

- 100: :analysis device
- 1: :measurement cell
- 11: :inlet
- 13: : outlet
- SP: :internal space
- 3: :LED light source
- 5: :detection unit
- 51: :optical filter
- 7: :introducing unit
- a-c: :gas port
- 9: :control unit
- 91: :arithmetic unit
- 93: :display unit
- 95: :input unit
- 97: :alarm generation unit
- Gr: :reference gas
- Gs: :sample gas
- I₁: :first current
- I₂: :second current
- L: :ultraviolet light
- Th: :predetermined threshold value

## Claims

1. An analysis device for analyzing an analysis target gas by using ultraviolet light, the device comprising:
a measurement cell to which the analysis target gas is introduced;
an LED light source configured to output the ultraviolet light toward the measurement cell; and
a control unit configured to control the LED light source, wherein
the control unit supplies a constant first current to the LED light source, and
when intensity of the ultraviolet light output from the LED light source supplied with the first current decreases to a predetermined threshold value or less, the control unit supplies the LED light source with a second current larger than the first current.

2. The analysis device according to claim 1, further comprising an input unit configured to receive a user's predetermined operation, wherein
when the predetermined operation is performed on the input unit, the control unit changes the current supplied to the LED light source from the first current to the second current.

3. The analysis device according to claim 1 or 2, further comprising an alarm generation unit configured to generate an alarm when the ultraviolet light output from the LED light source becomes a predetermined threshold value or less.

4. The analysis device according to any one of claims 1 to 3, further comprising an introducing unit configured to alternately introduce the analysis target gas and a reference gas into the measurement cell at a predetermined period.

5. An analysis method for analyzing an analysis target gas by using ultraviolet light, the method comprising:
introducing the analysis target gas into a measurement cell;
supplying a constant first current to an LED light source configured to output the ultraviolet light to output the ultraviolet light from the LED light source toward the measurement cell; and
supplying the LED light source with a second current larger than the first current, when intensity of the ultraviolet light output from the LED light source supplied with the first current decreases to a predetermined threshold value or less.

6. A program that causes a computer to perform an analysis method for analyzing an analysis target gas by using ultraviolet light, the analysis method comprising:
introducing the analysis target gas into a measurement cell;
supplying a constant first current to an LED light source configured to output the ultraviolet light to output the ultraviolet light from the LED light source toward the measurement cell; and
supplying the LED light source with a second current larger than the first current, when intensity of the ultraviolet light output from the LED light source supplied with the first current decreases to a predetermined threshold value or less.
